# EUROPÄISCHE PATENTSCHRIFT

(11) **EP 2 545 945 B1**
(45) Veröffentlichungstag und Bekanntmachung des Hinweises auf die Patenterteilung: **29.11.2023**
(21) Anmeldenummer: 12005107.3
(22) Anmeldetag: 10.07.2012
(51) Int. Cl.: A61L 27/04, A61L 27/56, A61L 27/58

(54) **Implantat, Bauteilset, Verfahren zur Herstellung eines Implantats und/oder eines Bauteilsets und Vorrichtung zur Herstellung eines Implantats und/oder eines Bauteilsets**
Implant, component set, method for manufacturing an implant and/or a component set and device for manufacturing an implant and/or a component set
Implant, kit de composant, procédé de fabrication d'un implant et/ou d'un kit de composant et dispositif de fabrication d'un implant et/ou d'un kit de composant

(30) Priorität: 11.07.2011 DE 102011107577
(43) Veröffentlichungstag der Anmeldung: 16.01.2013
(73) Patentinhaber: Meotec GmbH & Co. KG, 52074 Aachen (DE)
(72) Erfinder: Kopp, Alexander, 52062 Aachen (DE); Smeets, Ralf, 97892 Kreuzwertheim (DE)
(74) Vertreter: FARAGO Patentanwälte GmbH

(56) Entgegenhaltungen:
- WO-A1-97/36708
- DE-A1- 3 435 771
- DE-A1- 3 639 030
- US-A1- 2009 081 313

## Beschreibung

Die Erfindung betrifft ein Implantat, ein Bauteilset, ein Verfahren zur Herstellung eines Implantats und/oder eines Bauteilsets und eine Vorrichtung zur Herstellung eines Implantats und/oder eines Bauteilsets.

Implantate sind aus dem Stand der Technik zahlreich bekannt. Um fehlenden Knochen zu ersetzen und Frakturen zu fixieren werden Implantate seit langem eingesetzt. Beispielsweise Knochendefekte in der Kiefer- und Gesichtschirurgie, zum Beispiel in Folge von Unfällen, Missbildungen oder Tumorresektionen bedürfen ausgedehnter Rekonstruktionen. Diese medizinischen Maßnahmen sind mit hohen volkswirtschaftlichen Kosten verbunden und belasten das Gesundheitssystem.

Die Auswahl an Implantatwerkstoffen in der Medizintechnik ist aufgrund der geforderten biologischen Kompatibilität gering. Ansätze, fehlenden Knochen durch feste, das heißt belastbare Materialien zu ersetzen, können grob in zwei Gruppen unterteilt werden: Ein Ersatz durch resorbierbare und einen Ersatz durch nichtresorbierbare Implantate.

Aufgrund der bisher ungelösten Probleme beim Degradationsverhalten sowie den Zersetzungsprodukten von resorbierbaren Implantaten sind nichtresorbierbare Implantate aus chirurgischen Stählen oder Titan derzeitiger Standard. Titan weist gute Eigenschaften hinsichtlich Dauer, Festigkeit, Korrosionsbeständigkeit und Biokompatibilität auf.

Das Implantat bleibt jedoch ein Fremdkörper und muss nach einer gewissen Zeit aufgrund von Entzündungen, Reizungen oder Alterung entfernt werden. Die Revisionsoperation verursacht erneut hohe Kosten und erhöht wiederum das klinische Risiko. Zudem bedeuten weitere Operationen eine schwerwiegende Gesundheitsbelastung für den Patienten, lange Krankenhausaufenthalte und damit verbunden krankheitsbedingte Arbeitsausfälle, was einen nicht zu unterschätzenden wirtschaftlichen Schaden mit sich bringt.

Häufig kommt es zudem zu Komplikationen bis hin zu Refrakturen z. B. durch Lastabschirmung, bei der der umliegende Knochen degeneriert. Auch eine Lockerung des Implantats durch abrasives Mikrogleiten tritt auf. Auch hier sind weitere invasive Behandlungsmaßnahmen notwendig.

Letztendlich muss auch der Komfort für den Patienten berücksichtigt werden. Häufig klagen Patienten über Unbehagen, weil sich Implantate unter Sonneneinstrahlung erwärmen oder die Unterschiede der mechanischen Eigenschaften von Implantat und Knochen zu Problemen führen

Aus diesen Gründen wurden zahlreiche Versuche unternommen bioresorbierbare Implantate zu entwickeln, die vom Körper absorbiert und in natürlichen Knochen umgewandelt werden. Dies ist z.B. durch die Transplantation von patienteneigenen Knochen möglich. Dabei wird Knochen, z.B. aus der Hüfte entfernt und an andere Stelle eingesetzt. Allerdings bedingt dieser Vorgang einen weiteren chirurgischen Eingriff, die damit verbundenen Kosten und Risiken, sowie eine Schwächung des Knochens an der Entnahmestelle. Nicht zuletzt ist autologer Knochen nur begrenzt verfügbar und häufig kommt es durch den unterschiedlichen Aufbau des Knochens zu Zellsterben im unterversorgten Inneren größerer Transplantate.

Körperfremde Implantate aus resorbierbaren Werkstoffen wären dagegen beliebig verfügbar. Aufgrund der hohen Anforderungen hinsichtlich Biokompatibilität, Festigkeit und Degradationsverhalten sind solche Materialien jedoch kaum bekannt. Polyactide (PLA) finden in Form von Nahtmaterialien, Schrauben oder Platten bereits seit einiger Zeit Anwendung in der Medizintechnik. Sie sind biokompatibel und abbaubar, degradieren jedoch azide und weisen eingeschränkte mechanische Kennwerte auf. In Form von Folien oder Platten verschließt der dichte Werkstoff häufig Hohlräume, in denen Entzündungen und Nekrosen durch fehlende Transportmechanismen heranwachsen und zu starken Komplikationen führen.

Im Vergleich hierzu weisen biokompatible Magnesiumlegierungen fast optimale Eigenschaften auf. Magnesium ist als Mineral in fast allen Körperzellen vorhanden und seine Legierungen sind deutlich fester als Kunststoff. Die im Vergleich zu Titan geringe Festigkeit ist sogar erwünscht, da die mechanische Festigkeit und das Elastizitätsmodul der meisten Magnesiumlegierungen vergleichbar mit dem menschlichen Knochens ist. Magnesium als körpereigener Bestandteil erfüllt demnach aufgrund seiner uneingeschränkten Biokompatibilität und knochenähnlichen Festigkeit alle Voraussetzungen für einen Implantatwerkstoff. Trotz guter Voraussetzungen und der theoretischen Überlegenheit Magnesiums als Implantatwerkstoff, ist es auch in zahlreichen Forschungsbemühungen bislang nicht gelungen ein marktreifes Magnesiumimplantat zu entwickeln. Die spontan ablaufende Reaktion von Magnesium mit Wasser in einem feuchten Milieu führt zu einer Verschiebung des pH-Werts und zur Freisetzung von Wasserstoffgas. Die äußerst dynamische Umsetzung des Magnesiums führt deshalb zu massiver Blasenbildung (1 g Mg setzt 1,03 1 Wasserstoffgas frei). Die Folge sind Irritationen und Entzündungen sowie das frühzeitige Versagen des Implantats unter Last. Zudem kommt es im Inneren größerer Implantate zu vermehrtem Zellsterben durch fehlende Versorgung der Zellen.

Für einen Durchbruch von Magnesium als Implantatwerkstoff ist es deshalb notwendig, die Degradationskinetik so zu beeinflussen, dass dem umliegenden Gewebe ausreichend Zeit zur Verfügung steht, einwachsenden Knochen aufzubauen und die ansonsten biokompatiblen Degradationsprodukte abzutransportieren. Um dies zu realisieren wurden zahlreiche Projekte ins Leben gerufen, die das Ziel hatten den Abbau des Magnesiums durch Beeinflussung der Legierung und des Gefügezustands langsamer zu gestalten. Bisher ist es jedoch nicht gelungen, das Degradationsproblem durch die Werkstofffrage zu lösen.

Neuere Ansätze verfolgen das Ziel, über die Modifikation der Randzone im Rahmen einer mechanischen Verdichtung, die Resorptionsrate zu beeinflussen. Auch dieser Ansatz liefert bislang keine zufriedenstellenden Ergebnisse.

Ein weiterer Ansatz ist die Herstellung von Magnesiumschwämmen durch einen Ausgasungsprozess, bei dem chaotisch angeordnete Poren auf der Oberfläche das Einwachsverhalten von Gewebe begünstigen. Trotz guter Resultate diesbezüglich, löst sich die Schwammstruktur sogar überdurchschnittlich schnell auf. Zudem weist die ungeordnete Struktur eine geringere mechanische Stabilität auf. Vereinzelt führen überproportional hohe Porendichten in den Bauteilen zu vorzeitigem Versagen.

Ein neuer Ansatz zur Steuerung der Degradation ist die elektrochemische Umwandlung der Oberfläche. Versuche haben gezeigt, dass durch die Konversion der Oberfläche im Rahmen der Anodisation das Degradationsverhalten effektiv beeinflusst werden kann. Diese Entwicklung ist aufgrund der zuvor beschriebenen Problematik bei größeren Implantatvolumen derzeit jedoch auf filigrane Strukturen eingeschränkt.

Die DE 36 39 030 A1 betrifft Knochenimplantate, vorzugsweise aus Titan, wobei die plattenförmigen, gerollten oder geformten Implantate gerade rohrförmige Kanäle aufweisen. Die DE 36 39 030 A1 schweigt sich über Magnesiumimplantate aus und auch über eine Mikrostrukturierung der Implantate durch Oberflächenbeschichtung.

Die US 2009/081313 A1 offenbart biologisch abbaubare Magnesiumlegierungen für Knochenimplantate. Sie tragen vorzugsweise eine poröse Beschichtung, die z.B. die Biokompatibilität verbessert und den Abbau reguliert, z.B. verzögert. Eine Magnesiumoxid-Schicht kann durch anodische Oxidation in einem elektrolytischen Verfahren, z.B. in einem Bad aus Ammoniumhydroxid und Ammoniumhydrogenphosphat, aufgebracht werden. Die US '313 schweigt sich allerdings über eine Mikrostrukturierung der Oberfläche aus, wobei die Oberfläche eine Kalziumphosphatschicht und/oder eine Oxidschicht aufweist, bzw. eine Anodisation oder SLM-Behandlung beinhaltet.

Die WO 1997/36708 A1 offenbart eine Elektrode für die Funkenerosion zur Herstellung einer Struktur auf Implantaten, bei der die Elektrode das Negativ der angestrebten Struktur darstellt. Die WO ' 708 A1 offenbart hier keine Verwendung der Elektrode zur Herstellung eines Implantats mit Makro- und Mikrostruktur und auch nicht zur Herstellung einer Oberfläche, die eine Kalziumphosphatschicht und/oder eine Oxidschicht aufweist.

Die DE 34 35 771 A1 offenbart eine aus Knochenmaterial gewonnene Knochenmatrix mit Löchern von regelmäßigem bis unregelmäßigem Querschnitt mit Durchmessern von 0,25 -1,0 mm, die z.B. mittels Laser erhalten wurden. Die DE 34 35 771 A1 schweigt sich über Metallimplantate aus.

Aufgabe der vorliegenden Erfindung ist es daher, ein verbessertes Implantat zur Verfügung zu stellen.

Diese Aufgabe wird nach einem ersten Aspekt der Erfindung durch ein Implantat mit einem Implantatkörper aus Magnesium gelöst, wobei der Implantatkörper eine Makrostruktur aufweist, und bei welchem die Makrostruktur die Form von Kanälen aufweist, dadurch gekennzeichnet, dass eine Oberfläche des Implantats mikrostrukturiert ist, wobei die Oberfläche eine Kalciumphosphatschicht und/oder eine Oxidschicht aufweist, und/oder wobei die Oberfläche durch Anodisation behandelt ist, wodurch eine Oxidschicht mit regelmäßiger oder unregelmäßiger Oberflächenstruktur entsteht, die Poren aufweist, und/oder wobei die Makrostrukturierung der Kanäle in Form einer dreidimensionalen interkonnektierenden Kanalstruktur durch SLM hergestellt ist.

Unter Magnesium ist sowohl reines Magnesium als auch beschichtetes Magnesium zu verstehen. Auch resorbierbare Magnesiumlegierungen sollen erfasst sein.

Die Makrostruktur des Implantates bezieht sich auf seine grundlegenden äußeren Designmerkmale. Hierunter ist jedoch nicht die dreidimensionale Form des Implantates zu verstehen, sondern eine Strukturierung des Implantatskörpers in sich. Insbesondere mit bloßem Auge erkennbare Strukturen des Implantatkörpers sollen erfasst sein. Diese Strukturen können vorzugsweise regelmäßig sein.

Durch die Makrostrukturierung wird eine Verwendung von Magnesium mit seiner uneingeschränkten Biokompatibilität und knochenähnlichen Festigkeit als Implantatwerkstoff möglich.

Erfindungsgemäß weist die Makrostruktur die Form von Kanälen auf. Diese können durchgehend durch den Implantatskörper laufen. Sie können insbesondere in einem regelmäßigen Muster angeordnet sein und geradlinig verlaufen. Unregelmäßige Formen sind jedoch ebenfalls möglich.

Ein solches Implantatdesign hat zahlreiche Vorteile:

Erstens ist eine Versorgung des neugebildeten Knochengewebes mit Nährstoffen durch Kanäle möglich. Eine Nekrose im Inneren des Implantats wird vermieden.

Zweitens wird unter Umständen die gesamte Menge des vom Körper abzubauenden Magnesiums reduziert. Gleichzeitig weist das Implantat eine ausreichende Festigkeit auf, unter Umständen werden sogar eine bessere mechanische Festigkeit und Stabilität ähnlich wie bei einer Fachwerkskonstruktion erzeugt.

Drittens kommt es schließlich zu einer gesteigerten biochemischen Aktivität während des Knochenheilungsprozesses durch eine signifikante Vergrößerung der Oberfläche.

Dieses führt im Falle einer Beschichtung zu einem verbesserten Degradationsverhalten durch ein höheres Schichtvolumen.

Dadurch liegt die Resorptionsrate bei geeigneter Gestaltung im selben oder ähnlichen Geschwindigkeitsbereich wie die Neubildung des Knochens, so dass eine Lastabschirmung für eine kürzere Zeit oder unter Umständen gar keine Lastabschirmung während der Regenerationsphase notwendig ist und der Defekt im Idealfall weitgehend und eventuell sogar vollständig mit körpereigenem Knochen ausheilt.

Weitere Vorteile sind der Entfall der Revisionsoperation zur Entnahme des Implantats und das hiermit verbundene klinische Risiko. Auch besteht ein geringeres Entzündungs- und Verträglichkeitsrisiko durch das Verwenden körpereigener Stoffe. Auch der Tragekomfort für den Patienten erhöht sich. Durch den hohen gesellschaftlichen Bedarf aufgrund der demografischen Entwicklung trifft das Implantat den Nerv der Zeit und reagiert auf die Überlastung des medizinischen Systems. Neben einem reduzierten Risiko für den Patienten wird das Gesundheitssystem durch wegfallende Eingriffe signifikant entlastet.

Die Kanäle können in eine oder mehrere Koordinatenrichtungen verlaufen. Insbesondere können sie eindimensional oder zweidimensional verlaufen. Besonders bevorzugt ist, wenn die Kanäle in alle drei Achsrichtungen laufen. Hierdurch entsteht eine interkonnektierende Kanalstruktur, wie sie ähnlich auch im natürlichen Knochen gegeben ist. Es verbleibt ein gerüstartiger Implantatskörper um die Kanäle. Dadurch wird die Nährstoffversorgung des einwachsenden Knochens weiter verbessert.

Von Vorteil ist, wenn die Kanäle einen rechteckigen Querschnitt aufweisen. Insbesondere ein quadratischer Querschnitt ist möglich. Aber auch andere Querschnittsformen, wie beispielsweise ein runder oder elliptischer Querschnitt oder selbst asymmetrische Querschnittsformen, sind denkbar. Die Querschnittskontur der Kanäle kann weiterhin durch eine besondere Kontur überlagert werden, beispielsweise in Form einer Welligkeit oder Zacken.

Die Kanäle können eine Breite und/oder Höhe von zirca 100 bis 1500 Mikrometern, vorzugsweise von 300 bis 1000 Mikrometern, insbesondere vorzugsweise von 500 bis 700 Mikrometern, aufweisen. Gleichzeitig kann auch die Wandstärke der zwischen den Kanälen stehenbleibenden Stege in dieser Größenordnung variiert werden. Dadurch kann das Verhältnis von Masse zur Oberfläche des Implantats angepasst werden.

Erfindungsgemäß ist bei dem Implantat die freie Oberfläche des Implantats mikrostrukturiert. Unter Mikrostrukturierung ist hierbei eine Strukturierung der Oberfläche zu verstehen, die nicht mehr mit bloßem Auge erkennbar ist. Hierfür wird die Oberfläche des Magnesiums umgewandelt. Erfindungsgemäß erfolgt die Mikrostrukturierung der Oberfläche des Magnesiums durch ein Konversionsverfahren. Es entsteht eine korrosions- und verschleißfeste Schicht, die durch geeignete Wahl der Verfahrensparameter hinsichtlich ihrer Rauhigkeit und Porosität so variiert werden kann, dass eine verbesserte Interaktion mit umliegendem Gewebe und einwachsenden Knochenzellen stattfindet. Die optimalen Bedingungen für die Mikrostrukturierung können durch Biokompatibilitätstests überprüft werden. Insbesondere schützt die mikrostrukturierte Schicht auf der Oberfläche das korrosionsempfindliche Magnesium vor dem vorzeitigen Auflösen im feuchten Milieu des Körpers.

Dies geschieht aufgrund der höheren Beständigkeit der Schicht und des unter Umständen punktuellen Einsetzens von Schichtkorrosion, durch welche Fehlstellen erzeugt werden, die die Resorption des Grundmaterials steuern. Auf diese Weise kann durch die Wahl der Schichtparameter auch das Auflösungsverhalten des beschichteten Magnesium-Vollmaterials eingestellt werden.

Erfindungsgemäß weist die Oberfläche eine Kalciumphosphatschicht und/oder eine Oxidschicht auf. Bei der Kaliumphosphatschicht kann es sich zum Beispiel um Hydroxylapatit oder β-Trikalciumphosphat handeln. Bei der Oxidschicht kann es sich beispielsweise um Magnesiumoxid handeln. Eine solche entsteht typischerweise im Rahmen der Anodisation, wobei das Magnesium oxidiert wird. Die Oxidschicht ist fest mit dem Grundkörper verbunden. Sie verringert die Resorption. In einer Schicht können die oben genannten Stoffe auch gemischt vorliegen. Beliebige Schichten können auch hintereinander in beliebiger Kombination auftreten. Die zuletzt aufgetragene also äußerste Schicht kann auch aus einer organischen Beschichtung bestehen beziehungsweise kann derartige Bestandteile enthalten, so zum Beispiel aus üblichen Polymeren (z.B. PDLLA, PGLA) und/oder angekoppelten Molekülen zur Optimierung der Zellnische, hierbei seien insbesondere Zelladhäsionsmoleküle, Zytokine (Wachtums- oder Differenzierungsfaktoren) oder virale beziehungsweise non virale Genvektoren (u.a. Plasmide) genannt.

Diese Oxidschicht kann auch eine keramisierte Schicht sein, wie sie im Rahmen der plasmachemischen Anodisation entsteht. Diese ist noch stabiler und verringert die Resorption weiter.

Vorteilhafterweise weist die Oberfläche Poren mit einem Durchmesser von 0,1 bis 50 Mikrometern, vorzugsweise von 1 bis 10 Mikrometern, insbesondere vorzugsweise von 2 bis 5 Mikrometern auf. Dadurch kann die Oberflächeneigenschaft variiert werden. Eine verbesserte Interaktion mit umliegendem Gewebe und einwachsenden Knochenzellen findet statt.

Werden Mikrostrukturierung und eine geeignete Makrostrukturierung kombiniert, kann die Masse des Vollmaterials reduziert werden, während die Oberfläche und damit die Masse der Beschichtung vergrößert wird. Ist die Schicht nach längerem Einsatz im Körper weitestgehend oder vollständig aufgelöst, verbleiben lediglich kleine Inseln des Grundwirkstoffs, die bereits teilweise durch den neugebildeten Knochen gestützt werden und trotz erhöhter Resorption dann zu keinem wesentlichen Anstieg der Auflösungsrate mehr führen. Zusammenfassend hat die Kombination von Mikrostrukturierung und Makrostrukturierung zahlreiche Vorteile. Es ist bei geeigneter Ausgestaltung möglich, ein degradierbares Implantat aus Magnesium, das sich unabhängig von Größe und Form gleichmäßig auflöst, zu realisieren. Durch die körperfreundliche und gleichmäßig Dosierung der ansonsten biokompatiblen Zersetzungsprodukte werden die sonst üblichen Komplikationen verringert. Das Implantat erhält seine Stabilität im Idealfall lange genug, um dem umliegenden Knochen die notwendige Zeit für ein Ausheilen des Defektes bereitzustellen.

Ein weiterer Aspekt der Erfindung umfasst ein Verfahren zur Herstellung eines Implantats und/oder eines Bauteilsets für ein Implantat, wobei im ersten Schritt eine Makrostrukturierung und/oder eine Mikrostrukturierung erfolgt. Es kann also in einem ersten Schritt zunächst die Struktur erzeug werden, die mit dem bloßen Auge sichtbar ist. Auch die Erzeugung von nicht mehr mit bloßem Auge erkennbaren Strukturen kann alternativ oder kumulativ in einem ersten Schritt erfolgen.

Vorteilhafterweise erfolgt die Makrostrukturierung durch Funkenerosion (Electrical Discharge Machining, EDM) oder durch Electrochemical Machining, (ECM). So kann die nötige Formgenauigkeit und Oberflächenqualität sichergestellt werden.

Hierbei kann eine Elektrode mittels EDM oder ECM in eine oder mehrere, zum Beispiel drei, verschiedenen Ebenen in einen Magnesiumgrundkörper gesenkt werden. Die EDM- oder ECM-Bearbeitung bietet die notwendige Flexibilität hinsichtlich der Kanalgeometrie und ermöglicht das geforderte Aspektverhältnis.

Alternativ kann die Form auch durch ein generierendes Verfahren, beispielsweise Selective Laser Melting (SLM), hergestellt werden. In diesem Fall kann optional auch die äußere Gestalt des Implantats in einem Schnitt miterzeugt werden.

In einem nachgelagerten Schritt wird ansonsten aus dem Grundkörper auf Basis der klinischen Daten eines Patienten ein individuelles Implantat gefertigt. Dies geschieht entweder durch spanende Bearbeitung oder durch EDM oder ECM. Hierfür werden die Patientendaten aus bildgebenden Verfahren in Fertigungsdaten umgewandelt.

Alternativ wird die äußere Form durch Fügen von Bauteilen nach einem Baukastensystem angenähert oder hergestellt. Die Bauteile können bevorzugt als ein Bauteilset anlieferbar sein, welches diverse Grundformen beinhaltet, beispielsweise Ecken, Rundungen, Quader, Würfel, Stäbe usw.

Um der Form eine mechanische Stabilität zu verleihen, können die Bauteile aufeinander komplementär abgestimmte Hintergreifungen, Verhakungen, Verrastungen, Klemmungen, Verklebungen, Verschweißungen, oder generell form- oder kraftschlüssige Verbindemittel aufweisen. So kann durch Zusammenfügen im Bauteilset enthaltener Bauteile eine Makrostruktur für einen Grundkörper eines Implantats erstellt werden.

In einem nachgelagerten Schritt kann das Implantat beschichtet werden. Diese Beschichtung kann jedoch auch im ersten Schritt vor der Formgebung erfolgen.

Hierfür kann die Oberfläche durch Anodisation behandelt werden, wobei eventuell eine geordnete Porenstruktur entstehen kann. Durch die elektrochemische Reaktion an der Oberfläche des Implantats wird diese umgewandelt beziehungsweise oxidiert. Die Oxidschicht wächst hier nicht nur auf der Oberfläche selbst, sondern in das Volumen des Implantatkörpers hinein. Hierdurch entsteht eine ausgezeichnete Haftung. Die Oxidschicht wächst säulenförmig mit mittigen Poren, die sich jedoch nicht bis zum Grundmaterial erstrecken, sondern mindestens durch eine dünne Sperrschicht aus Oxiden vom Grundmaterial getrennt wird. Hierdurch wird die Resorptionsrate des beschichteten Implantats verringert.

Vorteilhafterweise kann die Oberfläche durch plasmachemische Anodisation behandelt werden. Hierdurch werden Oxidschichten erzeugt, die nochmals deutlich in ihrer Beständigkeit gesteigert sind. Plasmachemische Verfahren finden in speziellen Elektrolyten und bei wesentlich höheren elektrischen Feldstärken statt. Durch das hohe elektrische Feld, welches die Durchbruchspannung der sich bildenden Oxidschicht übersteigt, entstehen lokale Entladevorgänge. Diese resultieren in lokalem Plasma, wodurch mit hoher Temperatur und Druck die entstehende Oxidschicht modifiziert wird. Hierdurch entsteht eine sogenannte keramisierte Oberfläche und die Resorptionsrate des beschichteten Implantats wird weiter verringert.

Für eine plasmachemische Anodisation kann ein Elektrolyt verwendet werden, welcher mindestens folgendes aufweist:
a. Ammoniumhydroxid 25% (AH): 60 ml/l bis 400 ml/l
b. Diammoniumhydrogenphosphat (DAHP): 0,05 mol/l bis 0,2 mol/l und
c. Harnstoff (HS): 0 mol/l bis 1 mol/l
insbesondere jeweils etwa 48 % DAHP, 37 % HS und 15 % AH oder diese Bestandteile im selben Verhältnis zueinander der oben angegebenen maximal-Konzentrationen, gemessen als Volumen-% oder als Gewichts-%.

Von Vorteil ist, wenn der Elektrolyt einen pH-Wert im Bereich von 8-12, insbesondere von 9-11 aufweist.

Vorteilhafterweise kann für ECM und plasmachemische Anodisation ein gemeinsamer Elektrolyt, der für beide Verfahrensschritte geeignet ist, in einer ebenfalls für beide Schritte geeigneten Anlage verwendet werden. Hierbei befindet sich das Bauteil in dem Elektrolyten und ist während der gesamten Prozessdauer als Anode gepolt. Sowohl die Senkelektrode für die ECM als auch die Kathodenbleche für die Anodisation sind als Kathode gepolt. Während der elektrochemischen Bearbeitung findet die Gegenkontaktierung durch die Senkelektrode statt, während der Anodisation durch die Kathodenbleche. Lediglich die angelegte Spannung, Strom und der Signalverlauf entscheiden, ob die Oberfläche des Werkstücks anodisch umgewandelt wird oder ob die Oberfläche anodisch aufgelöst wird.

Ein weiterer Aspekt zu der Erfindung umfasst eine Vorrichtung zur Herstellung eines Implantats und/oder eines Bauteilsets und/oder zum Durchführen eines Verfahrens. Erfindungsgemäß ist die Elektrode für die EDM- und/oder ECM-Bearbeitung vorstrukturiert und weist unter Umständen die Form der Querschnittsfläche der Kanäle oder eine andere geometrische Gestalt auf.

Die Erfindung wird nachfolgend Anhand von Ausführungsbeispielen unter Bezugnahme auf die Zeichnungen näher erläutert.

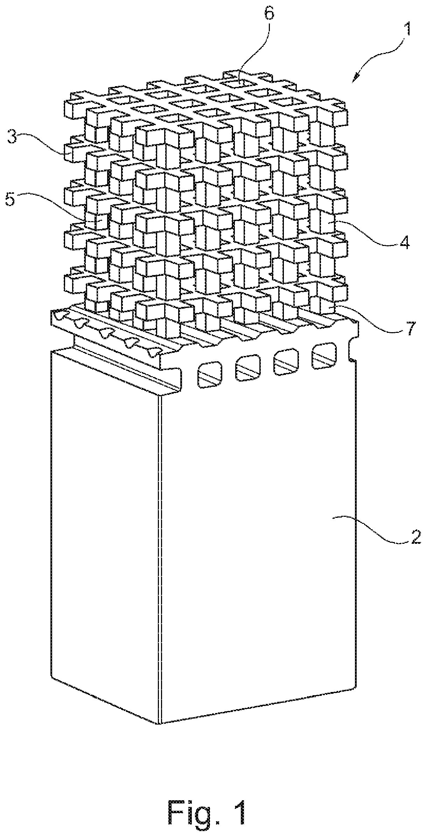

Hierin zeigen
- Figur 1: eine schematische Darstellung eines makrostrukturierten Grundkörpers.
- Figur 2: schematische Darstellungen möglicher Querschnittskonturen der Kanäle.
- Figur 3: schematische Darstellungen möglicher Kanalverläufe.
- Figur 4: einen Ausschnitt aus einer mikrostrukturierten Oberfläche.
- Figur 5: das schematische Abbauverhalten von beschichteten Magnesium-Implantaten und einem Implantatkonzept mit Mikro- und Makrostukturierung,
- Figur 6: ein schematisch dargestellter Fertigungsvorgang für ein Kieferimplantat, sowie

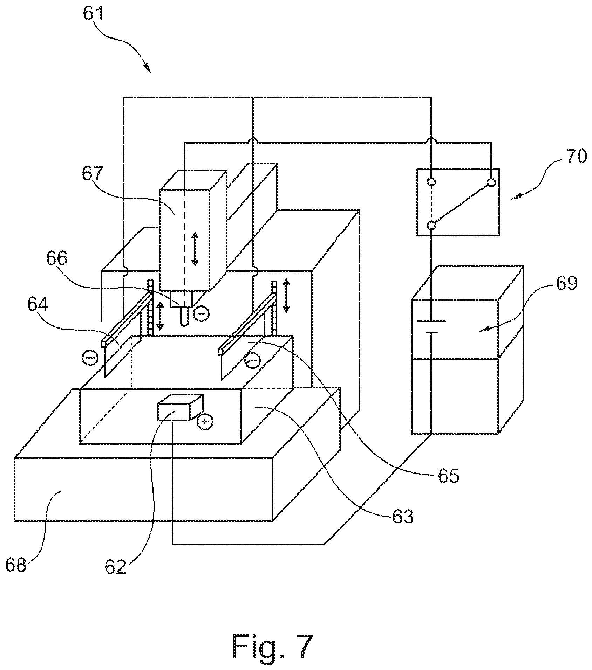

- Figur 7: eine schematisch dargestellte Fertigungsanlage zur ECM und plasmachemischen Anodisation.

Der Implantatkörper 3 weist eine Makrostruktur in Form von Kanälen 4, 5, 6 auf. Die Kanäle der Makrostruktur weisen eine Größe von 500 bis 700 Mikrometern auf. Dazwischen stehen Wände 7. Die Makrostrukturierung der Kanäle des erfindungsgemäßen Implantatskörpers ist in Form einer dreidimensionalen interkonnektierenden Kanalstruktur durch SLM hergestellt.

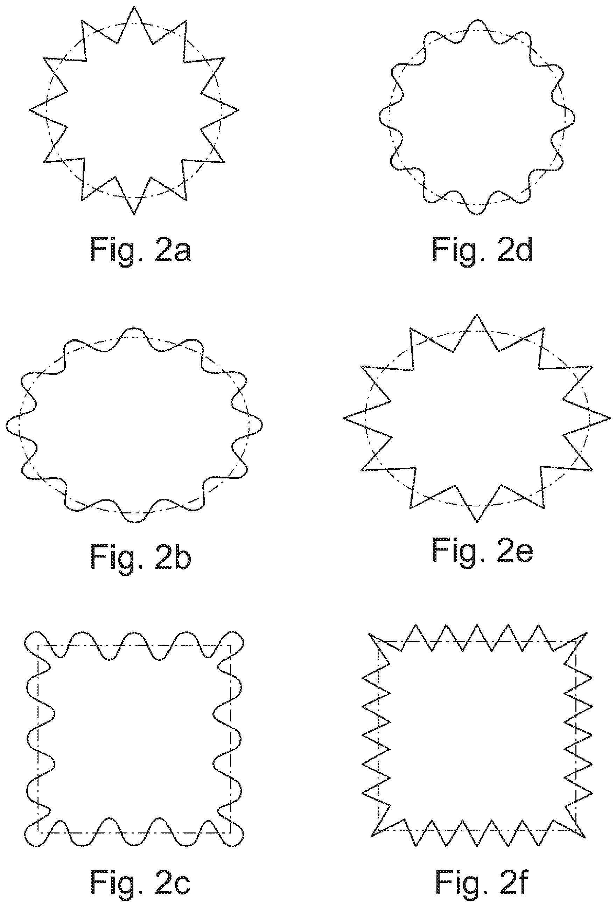

Die Querschnittskontur der Kanäle kann durch besondere Konturen überlagert werden. So zeigt Figur 2a einen kreisförmigen Querschnitt, der durch eine Welligkeit überlagert ist, Figur 2b einen elliptischen Querschnitt, der durch eine Welligkeit überlagert ist und Figur 2c einen rechteckigen Querschnitt, der durch eine Welligkeit überlagert ist. Figur 2d hingegen zeigt einen kreisförmigen Querschnitt, der durch eine Zackenkontur überlagert ist, Figur 2e einen elliptischen Querschnitt, der ebenfalls mit Zacken überlagert ist, und Figur 2f einen quadratischen Querschnitt ebenfalls mit Zackenüberlagerung.

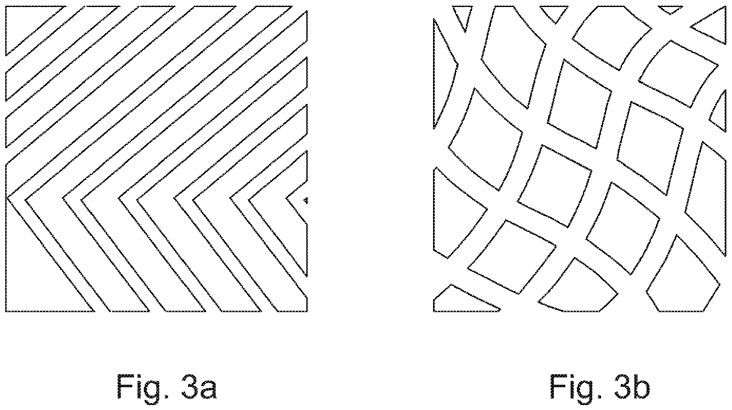

Als mögliche Kanalverläufe kommen wie in Figur 3a dargestellt beispielsweise zackenförmige Kanalverläufe in Betracht. Auch völlig unregelmäßige Kanalverläufe wie in Figur 3b dargestellt sind möglich.

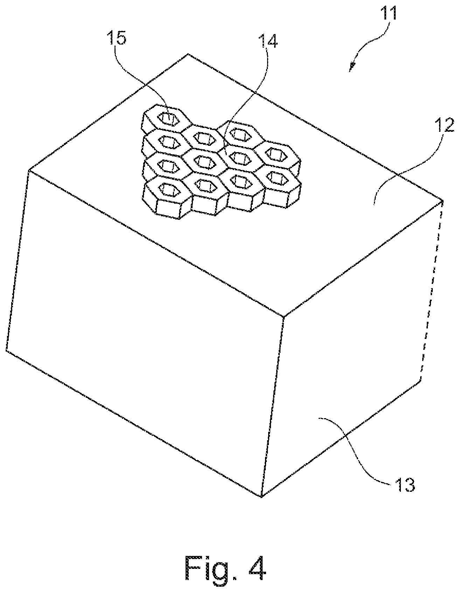

Für einen Implantatsgrundkörper mit einer mikrostrukturierten Oberfläche 11 wie in Figur 4 abgebildet wird die Oberfläche 12 des Grundkörpers 13 durch Anodisation behandelt. Hierdurch entsteht eine Oxidschicht 14 mit regelmäßiger Oberflächenstruktur beziehungsweise bei plasmachemischer Anodisation eine Oxidschicht unregelmäßiger Oberflächenstruktur (nicht abgebildet). Diese weist Poren wie beispielsweise 15 auf. Diese weisen eine Größe von 2 bis 5 Mikrometern auf.

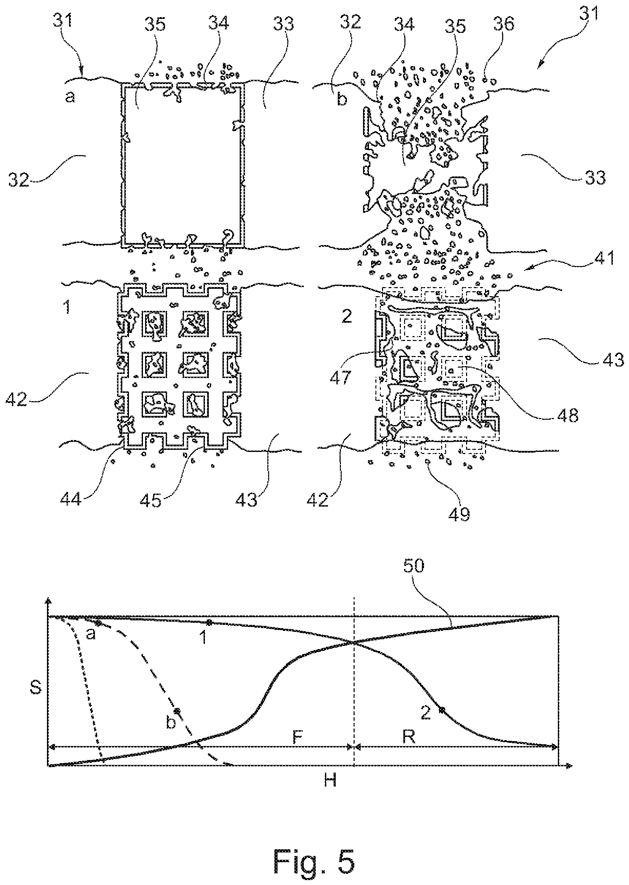

Figur 5 oberer Teil links bezeichnet mit a zeigt ein Implantat 31 aus beschichtetem Vollmaterial Magnesium am Beginn eines Einheilprozesses in zwei Knochenabschnitte 32 und 33. Das Implantat ist mit einer korrosions- und verschleißfesten Schicht 34 beschichtet, die den Magnesiumgrundkörper 35 vor dem vorzeitigen Auflösen im feuchten Milieu des Körpers schützt. Nach einem gewissen Zeitverlauf kommt es mit Fortschreiten des Einheilprozesses zu einer Schichtkorrosion und die Resorption des Grundmaterials setzt ein, Figur oberer Teil rechts bezeichnet mit b. Auf diese Weise kann zwar durch Wahl der Schichtparameter auch das Auflösungsverhalten des beschichteten Magnesium-Vollmaterials 35 positiv beeinflusst werden.

Da das Masseverhältnis von Grundmaterial gegenüber der Schicht zu groß ist, wird auf diese Weise nur der anfängliche Anstieg der Resorptionsrate begrenzt. Es verbleibt ein massiver Körper aus beschleunigt korrodierendem Magnesium, der frühzeitig versagt. Aufgrund des großen Volumens des Magnesiumvollmaterials kommt es zusätzlich zum frühzeitigen Implantatversagen zu verstärkter Wasserstoffbildung. Dieser tritt in Blasen wie beispielsweise 36 auf.

Aus diesem Grund wird die Mikrostrukturierung mit der voranstehend beschriebenen geeigneten Makrostrukturierung kombiniert, vergleiche Bild 2 in der Mitte. Wie hier dargestellt, ändert sich dieses Verhalten für ein makro- und mikrostrukturiertes Implantat 41. Auch hier wird der Einheilverlauf des Implantats 41 in zwei Knochenabschnitte 42 und 43 dargestellt, dabei zeigt der rechte mit 1 bezeichnete Abschnitt den Zustand zu Beginn des Einheilprozesses und der mit 2 bezeichnete Abschnitt den Zustand nach einem gewissen Zeitverlauf. Das Implantat 41 weist eine Makrostruktur in Form von Kanälen wie beispielsweise 44 auf. Auch eine Mikrostrukturierung der Oberfläche ist erfolgt, indem die Schicht 45 erzeugt wurde.

Durch das Einbringen von Kanälen wie 44 in allen Achsrichtungen entsteht eine dreidimensionale interkonnektierende Kanalstruktur, wie sie ähnlich auch im natürlichen Knochen gegeben ist. Auf diese Weise wird die Masse des Vollmaterials 46 reduziert, während die Oberfläche und damit die Masse der Beschichtung 45 vergrößert wird. Zudem ermöglichen die entstehenden Kanäle wie 44 eine verbesserte Nährstoffversorgung des einwachsenden Knochens 42 und 43 auch in den Kanälen. Ist die Schicht 45 nach längerem Einsatz im Körper im Wesentlichen aufgelöst, verbleiben lediglich kleine Inseln des Grundwerkstoffs wie beispielsweise 47, die bereits teilweise durch den neugebildeten Knochen wie beispielsweise an Stelle 48 gestützt werden und trotz erhöhter Resorption zu keinem wesentlichen Anstieg der Auflösungsrate führen. Insbesondere werden nur kleine Mengen von Wasserstoff in Blasen wie beispielsweise 49 freigesetzt.

Das Diagramm in Figur 5 unterer Teil zeigt das das Abbauverhalten von verschiedenen Implantatskonzepten als Funktion der Stabilität S gegen die Zeit, die die Heilphase H beträgt. Hierbei kann die Heilphase H in die Funktionsphase F und die Resorptionsphase R unterteilt werden. Die erste gepunktete Kurve repräsentiert reines Magnesium mit einer Reinheit von 99,99 %. Hier nimmt die Stabilität bereits am Beginn der Einheilphase sehr rasch ab. Eine ausreichende Funktionalität ist daher nicht gegeben. Die gestrichelte zweite Kurve zeigt das Abbauverhalten für ein Implantat aus beschichtetem Vollmaterial, wie es in Figur 5 im oberen Teil bereits beschrieben wurde. Hierin sind die Zeitpunkte a und b zugeordnet. Hier ist die Stabilität zwar bereits erhöht, das heißt ein Abbau erfolgt zu einem späteren Zeitpunkt in der Heilphase. Diese erfolgt jedoch immer noch zu frühzeitig in der Funktionsphase, als dass eine ausreichende Stabilisierung gewährleistet wäre. Die dritte durchgezogene Kurve zeigt das Abbauverhalten von einem Implantatkonzept mit Mikro- und Makrostrukturierung, wie es bereits in Figur 5 in der Mitte dargestellt ist. Auch hier sind die beiden Zeitpunkte 1 und 2 zu Beginn der Einheilphase und nach fortgeschrittener Einheilung bezeichnet. Hier ist deutlich, dass die Stabilität durch die Mikro- und Makrostrukturierung deutlich erhöht werden konnte. Ein Abbau erfolgt erst zu einem sehr späten Zeitpunkt im Rahmen der Resorptionsphase R. Über die Dauer der Funktionsphase F bleibt die Stabilität des Implantats fast vollständig erhalten. Viel mehr ist der Stabilitätsabbau mit dem Stabilitätsaufbau durch neu gebildeten Knochen synchronisiert. Dieser ist durch die Linie 50 repräsentiert. Während die Stabilität des Implantats in der Funktionsphase nur gering abnimmt, nimmt die Stabilität des neu gebildeten Knochens in der Funktionsphase F schon signifikant zu. In der Resorptionsphase R, in der die Stabilität des Implantats mit Mikro- und Makrostrukturierung rapide abnimmt, hat der neu gebildete Knochen fast seine vollständige Stabilität erreicht. Durch die aufeinander abgestimmte Aufnahme der Stabilität des Implantatkonzepts und der Zunahme der Stabilität des neu gebildeten Knochens entsteht im Idealfall bis zur vollständigen Resorption keine Phase der Instabilität.

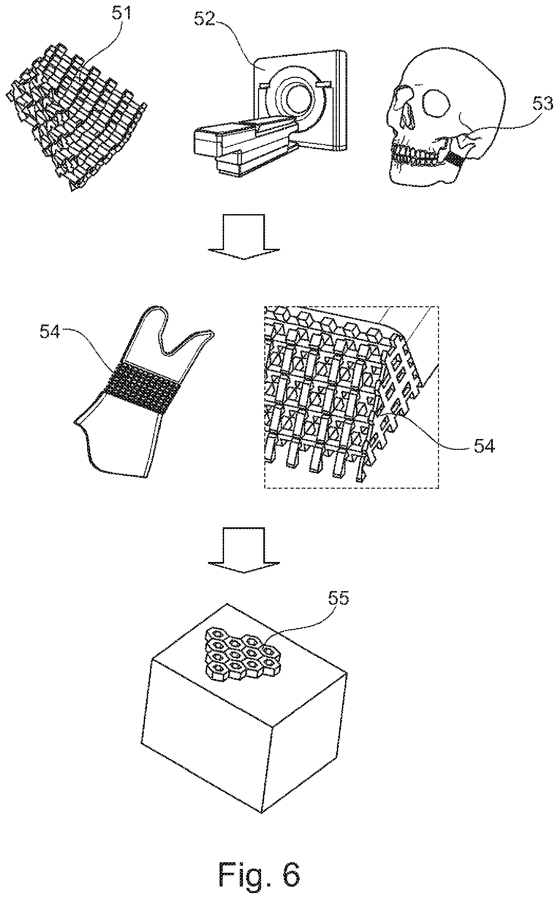

Um die gewünschte Struktur des Implantats herstellen zu können, wird wie in Figur 6 dargestellt zunächst der Grundkörper wie voranstehend beschrieben makrostrukturiert und so die Implantatstruktur 51 gefertigt. Anhand von klinischen Daten des Defektes, beispielsweise aus Computertomografie 52, werden CAD und CAM-Daten 53 generiert, und es, erfolgt eine Anpassung des Vormaterials. Hierdurch entsteht ein an den Defekt individuell angepasstes Implantat 54. Dieses wird dann in einem Beschichtungsprozess, wie schematisch in 55 dargestellt, mikrostrukturiert.

In einer Fertigungsanlage 61 kann sowohl eine ECM als auch eine plasmachemische Anodisation durchgeführt werden.

Bei einer ECM ist das Werkstück 62 als Anode gepolt und befindet sich in einem beliebigen, jedoch für beide Verfahrensschritte geeigneten Elektrolyten (nicht abgebildet) in einem Elektrolytbecken 63. Die Kathodenbleche 64 und 65 befinden sich nicht im Elektrolyten. Die Senkelektrode 66 an der Pinole 67, die als Kathode gepolt ist, wird in den Elektrolyten gesenkt. Aufgrund der angelegten Leistungsparameter, wie der Höhe der Stromdichte und der Spannung, sowie der Signalform, wie Rampen, Wechselstrom, unipolares Pulsen, bipolares Pulsen etc, wird das Werkstück 62 anodisch aufgelöst. Dafür werden Bahnen mit der Senkelektrode 66 gefahren, wodurch die äußere Form des Werkstücks 62 verändert wird.

Bei der Behandlung durch plasmachemische Anodisation ist das Werkstück 62 erneut als Anode gepolt und befindet sich in einem beliebigen, jedoch ebenfalls für beide Verfahrensschritte geeigneten Elektrolyten. Die Senkelektrode 66 an der Pinole 67 befindet sich nicht im Elektrolyten. Die Kathodenbleche 64, 65 werden in den Elektrolyten gesenkt. Aufgrund der angelegten Leistungsparameter wie der Höhe der Stromdichte und der Wechselspannung sowie der Signalform wie Rampen, unipolares Pulsen, bipolares Pulsen etc. wird die Oberfläche des Werkstücks 62 umgewandelt.

Zur Erhöhung der Stabilität weist die Fertigungsvorrichtung 61 ein Maschinengestell 68 auf. Eine Energieversorgung 69 mit einer Leistungsquelle stellt die notwendige Spannung über einen Schalter 70 sowohl für die Kathodenbleche 64, 65 als auch für die Senkelektrode 66 zur Verfügung. Weiter weist die Energieversorgung 69 eine Steuervorrichtung (nicht abgebildet) auf, mit der eine entsprechende Steuerung der Leistungsparameter möglich ist.

## Patentansprüche

1. Implantat mit einem Implantatkörper aus Magnesium oder einer Magnesiumlegierung, bei welchem der Implantatkörper eine Makrostruktur aufweist, und bei welchem die Makrostruktur die Form von Kanälen aufweist, **dadurch gekennzeichnet, dass** eine Oberfläche des Implantats mikrostrukturiert ist, wobei die Oberfläche eine Kalciumphosphatschicht und/oder eine Oxidschicht aufweist, und/oder wobei die Oberfläche durch Anodisation behandelt ist, wodurch eine Oxidschicht mit regelmäßiger oder unregelmäßiger Oberflächenstruktur entsteht, die Poren aufweist, und/oder wobei die Makrostrukturierung der Kanäle in Form einer dreidimensionalen interkonnektierenden Kanalstruktur durch SLM hergestellt ist.

2. Implantat nach Anspruch 1, **dadurch gekennzeichnet, dass** die Kanäle in eine oder mehrere Achsrichtungen verlaufen.

3. Implantat nach Anspruch 1 oder 2, **dadurch gekennzeichnet, dass** die Kanäle einen runden, elliptischen oder rechteckigen Querschnitt aufweisen.

4. Implantat nach einem der Ansprüche 1 bis 3, **dadurch gekennzeichnet, dass** die Kanäle eine Breite und/oder Höhe von ca. 100 bis 1500 µm, vorzugsweise von 300 bis 1000 µm, insbesondere vorzugsweise von 500 bis 700 µm, aufweisen.

5. Implantat nach einem der Ansprüche 1 bis 4, **dadurch gekennzeichnet, dass** die Oberfläche Poren mit einem Durchmesser von 0,1 µm bis 50 µm, vorzugsweise von 1 µm bis 10 µm, insbesondere vorzugsweise von 2 µm bis 5 µm, aufweist.

6. Implantat nach einem der vorstehenden Ansprüche, **dadurch gekennzeichnet, dass** es miteinander gefertigte Bauteile aus einem Bauteilset aufweist.

7. Bauteilset enthaltend Bauteile zum Zusammenfügen einer Makrostruktur für einen Grundkörper eines Implantats nach einem der vorstehenden Ansprüche.

8. Verfahren zum Herstellen eines Implantats nach einem der Ansprüche 1 bis 6 und/oder eines Bauteilsets nach Anspruch 7, **dadurch gekennzeichnet, dass** in einem ersten Schritt eine Makrostrukturierung und/oder eine Mikrostrukturierung eines Grundkörpers des Implantats erfolgt, wobei die Oberfläche des Implantats eine Kalciumphosphatschicht und/oder eine Oxidschicht aufweist, und/oder wobei die Oberfläche des Implantats durch Anodisation behandelt ist, wodurch eine Oxidschicht mit regelmäßiger oder unregelmäßiger Oberflächenstruktur entsteht, die Poren aufweist, und/oder wobei die Makrostrukturierung der Kanäle in Form einer dreidimensionalen interkonnektierenden Kanalstruktur durch SLM hergestellt ist.

9. Verfahren nach Anspruch 8, **dadurch gekennzeichnet, dass** eine Oberfläche durch Anodisation, insbesondere durch plasmachemische Anodisation, behandelt wird.

10. Verfahren zum Herstellen eines Implantats nach einem der Ansprüche 1 bis 6, vor allem Verfahren nach Anspruch 9, **dadurch gekennzeichnet, dass** für eine plasmachemische Anodisation ein Elektrolyt verwendet wird, welches mindestens folgendes aufweist:
a. Ammoniumhydroxid 25% (AH): 60 ml/l bis 400 ml/l
b. Diammoniumhydrogenphosphat (DAHP): 0,05 mol/1 bis 0,2 mol/l und
c. Harnstoff (HS): 0 mol/l bis 1 mol/l
insbesondere jeweils etwa 48 % DAHP, 37 % HS und 15 % AH oder diese Bestandteile im selben Verhältnis zueinander der oben angegebenen Maximal-Konzentrationen, gemessen als Volumen-% oder als Gewichts-%.

11. Vorrichtung zum Durchführen eines Verfahrens nach einem der Ansprüche 9 oder 10, wobei die Vorrichtung eine Elektrode aufweist, die für die Electrical Discharge Machining (EDM)-Bearbeitung oder Electrochemical Machining (ECM)-Bearbeitung vorstrukturiert ist und in eine oder mehrere verschiedene Ebenen des Magnesiumgrundkörpers gesenkt werden kann.

## Claims

1. An implant with an implant body made of magnesium or magnesium alloy, in which the implant body has a macrostructure, and in which the macrostructure has the form of channels, **characterized in that** a surface of the implant is microstructured, whereby the surface has a calcium phosphate layer and/or an oxide layer, and/or whereby the surface is treated through anodization, through which an oxide layer with a regular or irregular surface structure that has pores arises, and/or whereby the macrostructuring of the channels is produced in the form of a three-dimensional interconnecting channel structure by means of selective laser melting (SLM).

2. An implant according to Claim 1, **characterized in that** the channels run in one or more axial directions.

3. An implant according to Claim 1 or 2 **characterized in that** the channels have a round, elliptical, or rectangular cross-section.

4. An implant according to one of Claims 1 to 3 **characterized in that** the channels have a width and/or height of approximately 100 to 1500 µm, preferentially of 300 to 1000 µm, especially preferentially of 500 to 700 µm.

5. An implant according to one of Claims 1 to 4 **characterized in that** the surface has pores with a diameter of 0.1 µm to 50 µm, preferentially of 1 µm to 10 µm, especially preferentially of 2 µm to 5 µm.

6. An implant according to one of the preceding claims, **characterized in that** it has components manufactured together from one component set.

7. A component set containing components for a macrostructure for a base body of an implant according to one of the preceding claims.

8. A process for manufacturing an implant according to one of the Claims 1 to 6 and/or a component set according to Claim 7, **characterized in that** in a first step macrostructuring and/or microstructuring of a base body of the implant takes place, whereby the surface of the implant has a calcium phosphate layer or an oxide layer, and/or whereby the surface of the implant is treated through anodization, through which an oxide layer with a regular or irregular surface structure that has pores arises, and/or whereby the macrostructuring of the channels is produced in the form of a three-dimensional interconnecting channel structure by means of SLM.

9. A process according to Claim 8, **characterized in that** a surface is treated with anodization, specifically with plasmachemical anodization.

10. A process for manufacturing an implant according to one of the Claims 1 to 6, above all a process according to Claim 9, **characterized in that** for plasmachemical anodization, an electrolyte is used, which exhibits at least the following:
a. Ammonium hydroxide 25% (AH): 60 ml/l to 400 ml/l
b. Diammonium hydrogen phosphate (DAHP): 0.05 mol/1 to 0.2 mol/l and
c. Urea (HS): 0 mol/l to 1 mol/l
in particular, about 48% DAHP, 37% HS and 15% AH each or these components in the same ratio to one another as in the maximum concentrations indicated above, measured as % by volume or % by weight.

11. A device for performing a process according to one of the Claims 9 or 10, whereby the device has an electrode that is prestructured for electrical discharge machining (EDM) processing or electrochemical machining (ECM) processing and can be lowered into one or more different levels of the magnesium base body.

## Revendications

1. Implant avec un corps d'implant en magnésium ou un alliage de magnésium, pour lequel le corps d'implant présente une macrostructure, et pour lequel la macrostructure présente la forme de canaux, **caractérisé en ce qu'**une surface de l'implant est microstructurée, dans lequel la surface présente une couche de phosphate de calcium et/ou une couche d'oxyde, et/ou dans lequel la surface est traitée par anodisation, ce qui permet de produire une couche d'oxyde avec une texture surfacique régulière ou irrégulière présentant des pores, et/ou dans lequel la macrostructuration des canaux sous la forme d'une structure de canal interconnectée tridimensionnelle est fabriquée par dispositif SLM.

2. Implant selon la revendication 1, **caractérisé en ce que** les canaux s'étendent dans une ou plusieurs directions axiales.

3. Implant selon la revendication 1 ou 2, **caractérisé en ce que** les canaux présentent une section transversale ronde, elliptique ou rectangulaire.

4. Implant selon l'une quelconque des revendications 1 à 3, **caractérisé en ce que** les canaux présentent une largeur et/ou hauteur d'environ 100 à 1500 µm, de préférence de 300 à 1000 µm, de façon particulièrement préférentielle de 500 à 700 µm.

5. Implant selon l'une quelconque des revendications 1 à 4, **caractérisé en ce que** la surface présente des pores ayant un diamètre de 0,1 µm à 50 µm, de préférence de 1 µm à 10 µm, de façon particulièrement préférentielle de 2 µm à 5 µm.

6. Implant selon l'une quelconque des revendications précédentes, **caractérisé en ce qu'**il présente des composants assemblés provenant d'un kit de composants.

7. Kit de composants contenant des composants relatifs à une macrostructure pour un corps de base d'un implant selon l'une quelconque des revendications précédentes.

8. Procédé de fabrication d'un implant selon l'une quelconque des revendications 1 à 6 et/ou d'un kit de composants selon la revendication 7, **caractérisé en ce que** dans une première étape une macrostructuration et/ou une microstructuration d'un corps de base de l'implant a lieu, dans lequel la surface de l'implant présente une couche de phosphate de calcium et/ou une couche d'oxyde, et/ou dans lequel la surface de l'implant est traitée par anodisation, ce qui permet de produire une couche d'oxyde avec une texture surfacique régulière ou irrégulière présentant des pores, et/ou dans lequel la macrostructuration des canaux sous la forme d'une structure de canal interconnectée tridimensionnelle est fabriquée par dispositif SLM.

9. Procédé selon la revendication 8, **caractérisé en ce qu'**une surface est traitée par anodisation, en particulier par anodisation plasma-chimique.

10. Procédé de fabrication d'un implant selon l'une quelconque des revendications 1 à 6, notamment procédé selon la revendication 9, caractérisé en qu'une anodisation plasma-chimique met en oeuvre un électrolyte présentant au moins les éléments suivants :
a. Hydroxyde d'ammonium 25 % (AH) : 60 ml/l à 400 ml/l
b. Hydrogénophosphate de diammonium (DAHP) : 0,05 mol/1 bis 0,2 mol/l et
c. Urée (HS) : 0 mol/l à 1 mol/l
en particulier respectivement environ 48 % de DAHP, 37 % de HS et 15 % de AH ou ces composants dans le même rapport entre eux des concentrations maximales indiquées ci-dessus, mesurées en % de volume ou en % de poids.

11. Dispositif de réalisation d'un procédé selon l'une des revendications 9 ou 10, dans lequel le dispositif présente une électrode qui est préstructurée pour l'usinage par électroérosion (« Electrical Discharge Machining » EDM) ou l'usinage électro-chimique (« Electrochemical Machining » ECM) et peut être enfoncée dans un ou plusieurs plans différents du corps de base en magnésium.
